(19) Europäisches Patentamt
European Patent Office
Office européen des brevets

(11) Publication number: **0 061 541**
**A1**

(12) # EUROPEAN PATENT APPLICATION

(21) Application number: 81301341.4

(22) Date of filing: 27.03.81

(51) Int. Cl.[3]: G 01 N 33/50
G 01 N 33/80

(43) Date of publication of application:
06.10.82 Bulletin 82/40

(84) Designated Contracting States:
CH DE FR GB IT LI

(71) Applicant: BIOSPECIA LIMITED
47, Ealing Road
Wembley Middlesex(GB)

(72) Inventor: Sharma, Dr, Yash
2766, January Court
Falls Church Virginia 22063(US)

(74) Representative: Dixon, Donald Cossar et al,
Gee & Co. Chancery House Chancery Lane
London WC2 1QU(GB)

(54) **Immunological analysis and a biochemical agent therefor.**

(57) A biochemical agent is described for facilitating detection of immunological agglutination reactions in liquid suspensions, in particular red blood cell suspensions.

The reagent comprises from 0.1 to 1 g/l of a protein-compatible, water soluble, nonionic surface-active polyoxyalkylene ether or ester, and from about 0.1 to about 1.0 g/l of a protein-compatible water-soluble anionic detergent, dissolved in a buffered isotonic aqueous saline solution. When added to an agglutination reaction mixture, the biochemical agent increases the sensitivity of the agglutination reaction and promotes fast settling out of agglutinated matter from the reaction mixture, whilst non-agglutinated matter is maintained in suspension and can easily be computerised.

An apparatus particularly for use in blood grouping, comprises a loading station (10) for identified carrier racks (18) of test tubes (19), a dispenser (11) for antisera, a mixer (13), a photometric reader (16), an unloading station (17) and a print out unit (26).

./...

EP 0 061 541 A1

Croydon Printing Company Ltd.

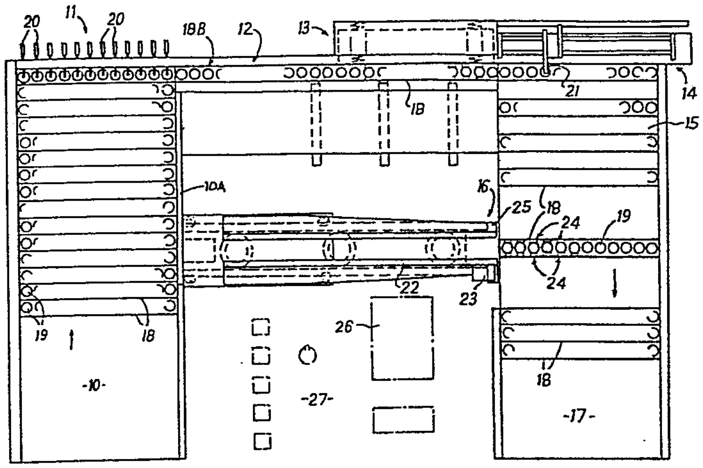
20
11
20
18B
12
13
21
14
18
15
10A
16
25
18
24
19
24
22
23
26
19
18
-10-
-27-
18
-17-

# IMMUNOLOGICAL ANALYSIS AND A BIOCHEMICAL AGENT THEREFORE

This invention relates to immunological analysis and, more specifically, to a biochemical agent for use in such analysis, to a method of detecting immunological agglutination, and to an apparatus for automating said method. The invention has particular application to blood-grouping.

Human bloods have different antigenic and immune properties, and the four major blood groups are distinguished from each other by the presence or absence in the red blood cells of different, but related, antigens. Blood of group A contains only antigen A, blood of group B contains only antigen B, blood of group O contains neither antigen A nor antigen B, and blood of group AB contains only antigen AB. There is also a rare blood group U.

The presence in the cells of the antigens which are capable of immunological reaction with their respective anti-bodies, makes the cells susceptible to agglutination; these antigens are therefore called agglutinogens. When an antigen is not present in the red blood cells, the respective antibodies, called agglutinins, develop in the blood plasma. Thus, for example, blood of group A contains anti-B antibodies in its serum, and group O blood contains both anti-A and anti-B agglutinins. If bloods are mismatched, so that anti-A or anti-B antibodies are mixed with red blood cells containing A or B antigens

respectively, the red cells will agglutinate due to the immunological antigen/antibody reaction. This causes the cells to clump and thus block blood vessels, with usually fatal results.

In addition to the O-A-B blood system, there are other factors which distinguish between different bloods, and these too are important in blood matching prior to transfusion. Primarily, different bloods are distinguished by the presence of Rh-agglutinogens which render a blood Rh-positive or Rh-negative. It can also be useful to consider platelets which influence blood clotting.

Accordingly, in order to avoid blood mismatching in blood transfusion, it is necessary to determine the blood group of the recipient and of the blood donor. Blood grouping is routine in medical laboratories and in blood banks, and there is a need for fast, simple and reliable blood-grouping method and apparatus.

Essentially, blood grouping is effected by contacting small amounts of a suspension of the red blood cells with known antisera, e.g. anti-serum A (containing agglutinin A) and anti-serum B (containing agglutinin B), and observing whether or not agglutination occurs.

Blood grouping and related immunological tests are still being carried out manually, usually by the slide technique in which blood is diluted with saline to obtain a suspension of red blood cells, and a drop of the saline suspension is mixed with a drop of the antiserum directly on a slide; after allowing several minutes for agglutination to take place, the slide is observed either by the naked eye or under a microscope

to determine whether or not the cells have clumped. According to a more reliable, now commonly used, method the red cell suspension and the antiserum are mixed in a small test tube and the mixture is centrifuged for at least 30 to 60 seconds. Whether or not agglutination has taken place is then examined by the naked eye whilst gently agitating the deposit in the tube, or by transferring the deposit onto a slide for microscopic examination.

More recently, devices for automating blood grouping procedures have been proposed, as, for example, in U.S. Patents Nos. 3,334,018 and 3,624,223 and Nos. 3,432,268, 4,104,031 and 4,130,395. The various prior devices for automated blood grouping comprises assemblies of a large number of specialized components and, accordingly, are costly to produce and are bulky; also, it has not been possible, either in prior manual or automated processes of blood grouping, to produce sufficiently reliable results in short periods of time, and most, if not all, processes are routinely duplicated and/or repeated. This is, of course, of great importance both for emergency situations and also for mass blood grouping which is envisaged to meet future plans for medical safety and also security purposes.

A reagent for promoting insolubilisation and comprising essentially polyethylene glycol and ethylene oxide is proposed in U.S. Patent No. 4,148,896, but this reagent is not suitable for use in analyses such as blood grouping where the antigens or antibodies are bound to effectively solid particles, i.e. red cells.

It is an object of the present invention to provide an improved biochemical agent which facilitates the recognition of immunological agglutination reactions in immunological tests, particularly in blood grouping methods which may be carried out either substantially manually or in at least partly automated procedures.

Further objects are to provide an improved method and apparatus for detecting agglutination of red blood cells in a liquid suspension, in which the disadvantages of the prior art are at least reduced.

According to the present invention, there is provided a biochemical agent for use in immunological analysis, to improve the detectability of immunological agglutination reactions in liquid suspensions, said agent comprising a solution of from about 0.1 to about 1.0 g/l of a nonionic surface-active agent selected from the group consisting of water-soluble protein-compatable polyoxyalkylene ethers and esters, and from about 0.1 to about 1 g/l of a water-soluble protein-compatable anionic detergent, in an aqueous isotonic buffered solution having a pH value of from about 6.5 to about 8.0, the solution exhibiting an osmolarity of from about 300 to about 400 milliosmol/kg, a sodium content of from about 125 to 225 milliequivalents, and a potassium content of from about 3.5 to 7.5 milliequivalents per litre.

In further accordance with the present invention, there is provided a method for detecting immunological agglutination in a liquid suspension, the method comprising:-

a) mixing a liquid suspension susceptible of comprising agglutinogen-containing agglutinable matter suspended therein, with a reagent containing agglutinins

reactive towards the agglutinogen content of the agglutinable matter to obtain a reaction mixture;

b) allowing a period of time suitable for an immunological reaction to take place;

c) mixing the reaction mixture with the biochemical agent as defined above to obtain a diluted reaction mixture; and,

d) examining the diluted reaction mixture for un-agglutinated matter suspended therein.

The biochemical agent is particularly useful as an adjuvant in, and the said method is particularly applicable to blood grouping, and, in further accordance with the present invention, an improved method of blood grouping comprises mixing a sample of the blood with antiserum, allowing a suitable period of time, e.g., between about 1 and about 10 minutes, for an immunological reaction to take place in the reaction mixture, and, subsequently, mixing the reaction mixture with an amount of preferably between about 10 and about 20 times its volume of the biochemical agent, to obtain a diluted reaction mixture. In the resulting diluted reaction mixture, agglutinated cells are substantially settled out and non-agglutinated cells are substantially retained in suspension. Accordingly, a simple visual inspection of the diluted reaction mixture, or a nephlometric detection of the suspended subject matter therein, is sufficient to give a reliable determination of whether or not the red cells of the blood sample are agglutinated by the given antiserum, and thus to determine the blood group.

In further accordance with the present invention, there is provided an apparatus for the automated detection of immunological agglutination in a liquid suspension, the apparatus comprising:

a) A loading station for receiving a carrier for a plurality of test tubes or like receptacles, each containing a sample of the liquid suspension;

b) a dispensing unit for dispensing a required amount of reagent liquid into each of the test tubes in the carrier;

c) a mixing unit;

d) a second dispensing unit for dispensing a metered amount of agent liquid into each of the test tubes;

e) a reading unit comprising a photometric system to pass beams of light through the test tubes and light-sensitive means for sensing the light transmitted through the test tubes;

f) an unloading station for discharging the test tube-containing carriers; and,

g) transport means for transporting the carriers from the loading station through the dispensing units, the mixing station, and the reading unit, to the unloading station.

In the primary application of the invention, in blood matching, the first dispensing means have individual connections to the test tubes from storage containers of individual reagents which contain agglutinins reactive to the agglutinogen-content of the agglutinable matter suspended in the respective liquid suspensions, and the second dispensing means are connected to at least one storage container of an agglutination detection adjuvent such as the biochemical agent according to the present invention. In blood grouping, the reagents contained in the first storage containers are antisera.

The use of the biochemical agent according to the present invention increases the sensitivity of the agglutinable matter in the test sample, thus allowing reduction in the amount of reactants used per test and also promoting clarity of results to minimise the risk that a weak positive reaction is not detected and, thus, also minimising the requirement for the use of an enzyme preparation. The biochemical agent also accelerates the agglutination reactions and promotes substantially complete settling out of agglutinated matter, primarily cell-agglutinations, while maintaining substantially all non-agglutinated matter in suspension for a long period of time, which can be several hours.

Furthermore, the use of the biochemical agent may eliminate the need for washing red blood cells and/or preparing diluted suspensions thereof prior to analysis, and eliminate the need for centrifuging and also for microscopical examination.

Embodiments of the invention will now be described, by way of example, and reference will be made to the accompanying drawing which is a schematic plan view showing an apparatus primarily for use in blood grouping.

It has been found that the sensitivity of immunological agglutination reactions can be improved and the distinction between agglutinated matter and non-agglutinated suspended matter can be largely facilitated by adding to the reaction mixture of the liquid suspension to be examined for agglutinable matter suspended therein and the reagent susceptible of causing an agglutination reaction, a biochemical agent which is a solution of a water soluble protein-compatable nonionic surface-active agent and a water soluble protein-compatable anionic detergent in an aqueous isotonic solution as defined above.

The isotonic aqueous buffered solution suitably is an isotonic saline solution comprising sodium chloride, potassium chloride and an alkali phosphate buffer which is buffered to a pH of from about 6.5 to about 8.0 preferably of from about 7.2 to about 7.4.

The sodium content of the biochemical agent is between about 125 and about 225, preferably between about 150 and about 200, most preferably about 175, e.g., 175± 10, milliequivalents of sodium per liter.

The potassium content of the biochemical agent is between about 3.5 and about 2.5, preferably between about 4.3 and about 6.3 milliequivalents of potassium per liter.

The osmolarity of the biochemical agent is from about 300 to about 400 preferably from about 330 to about 350 milliosmol/kg.

A high degree of clearness and substantial absence of any suspended particles are required in the biochemical agent, e.g., the nephlometric background count should be less than 100 particles/ml.

Both the nonionic surface-active agent and the anionic detergent should be highly water soluble and must be

compatable with proteins. In particular, they must be free of complex-forming or precipitating effects toward the proteinous matter in the agglutination reaction mixture.

The nonionic surface-active agents within the biochemical agent of the present invention include polyoxyalkylene ethers and esters.

The polyoxyalkylene part of the compounds may contain recurring oxyethylene alone or in admixture with oxypropylene units and preferably is a polyoxyethylene residue of sufficient chain length to provide water solubility to the compounds, e.g., a polyoxyethylene residue containing from about 6 to about 25, preferably from about 10 to about 25 oxyethylene units.

Suitable polyoxyalkylene ethers include ethers of the polyoxyalkylene with straight or branched chain alkyl or alkenyl alcohols containing between about 6 and about 25 preferably between about 9 and about 20 carbon atoms, such as nonyl alcohol trimethylnonyl alcohol, lauryl alcohol, stearyl alcohol, palmityl alcohol, oleyl alcohol or cetyl alcohol, or with phenyl alkyl alcohols containing up to 25 carbon atoms such as nonylphenyl alcohol; and ethers of the polyoxyalkylene with sorbitan fatty acid esters, e.g., sorbitan mono- and diesters of saturated and unsaturated fatty acids containing from about 12 to about 20 carbon atoms, such as sorbitanmonolaurate, sorbitanmonopalmate, sorbitan monostearate, sorbitan monooleate, sorbitan dioleate.

Suitable polyoxyalkylene esters include esters of the polyoxyalkylene with fatty acids containing from about 12 to about 20 carbon atoms such as lauric acid palmitic acid, stearic acid or oleic acid.

Suitable polyoxyethylene ether and esters can be represented by the following formula I

$$H(CH_2-CH_2O)_nR$$

wherein n represents a number of from about 6 to about 25 and R represents alkyl or alkenyl containing from about 6 to about 25 carbon atoms, phenylalkyl containing from 7 to about 25 carbon atoms, alkylcarbonyl or alkenylcarbenyl containing from about 12 to about 20 carbon atoms, or a sorbitan mono or diester of an alkylcarboxylic or alkenylcarboxylic acid containing from about 12 to about 20 carbon atoms.

Among the foregoing, polyoxyethylene ethers and esters, compounds wherein R is alkyl containing 12 to 16 carbon atoms and n is a number from about 20 to about 25, in particular polyoxyethylene-23-laurylether, are preferred.

Any conventional anionic detergent which is sufficiently water soluble and protein-compatable can be used within the biochemical agent.

Suitable anionic detergents include alkali, e.g., sodium or potassium, ammonium, lower alkyl amine, e.g., triethylamine and lower hydroxyalkylamine, e.g., triethanolamine, salts, preferably sodium salts, of organic derivatives of sulphur-oxygen acids such as alkyl-, alkylether- and alkylphenyl sulfates and sulfonates, and organic derivatives of phosphor-oxygen acids such as alkyl-, alkylether- or alkylphenyl phosphates and phosphonates.

Particularly suitable organic sulfates include alkylsulfates of the formula II and alkylether sulfates of the formula III

$$R\text{-}OSO_3^- \quad \text{(II)} \qquad RO(CH_2\text{-}CH_2O)_xSO_3^- \quad \text{(III)}$$

wherein R is alkyl containing from about 6 to about 20 carbon atoms, in particular from about 12 to about 18 carbon atoms, and x represents a number of from 1 to about 9 preferably from about 3 to about 5. Examples of suitable alkyl groups R include lauryl, myristyl, stearyl, 2-ethylhexyl or cetyl. Laurylsulfate and laurylether sulfates

containing 3 to 5 oxyethylene units are preferred.

Suitable organic sulfonates include alkane sulfonates of the formula IV and alkylbenzene sulfonates of the formula V

$R-SO_3^-$ IV and 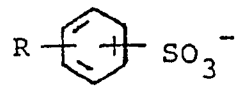 V

wherein R is as defined above.

Suitably the biochemical agent comprises from about 0.1 to about 1.0, preferably from about 0.5 to about 1.0 g/l of the nonionic surfactant and from about 0.1 to about 1, preferably from 0.5 to 1.0 g/l of the anionic detergent.

The present invention provides an important improvement in immunological test procedures for detecting agglutination reactions.

According to the method of the present invention for detecting immunological agglutination in a liquid suspension, a liquid suspension susceptible of containing agglutinable matter suspended therein and a reagent susceptible of containing agglutination-causing components are reacted with each other in a conventional manner, the resulting reaction mixture is mixed with the biochemical agent and the resulting diluted reaction mixture is examined for non-agglutinated matter retained in suspension therein.

Addition of the biochemical agent to the agglutination reaction mixture provides several important advantages:

a) the sensitivity of the agglutinable matter and the agglutination-causing components in the reaction mixture is increased leading to a substantially complete agglutination, even in the case of an only weak activity per se of the agglutinating components of the reaction mixture;

b) the speed of the agglutination reaction is accelerated;

c) the need of a purifying and/or diluting

pretreatment of the liquid suspension of the agglutinable matter can be eliminated. It is believed that the foregoing advantages are largely due to the presence of the detergent in the biochemical agent, e.g., a washing effect of the detergent on the suspended particles of agglutinable matter;

d) settling out of agglutinated matter is promoted whereas non-agglutinated matter is maintained substantially completely in suspension for a prolonged period of time, e.g., for a period of up to several hours. This advantageous stabilization of the suspension of suspended non-agglutinated matter is believed to be largely due to the effect of the nonionic surfactant in the biochemical agent;

e) due to the substantially complete separation of settled out agglutinated matter from suspended non-agglutinated matter in the diluted reaction mixture containing the biochemical agent, determination of whether or not an agglutination has taken place can readily be made by visual inspection or by nephlometric inspection of the diluted reaction mixture, without the need of any separation-enhancing pretreatment, e.g., centrifugation of the mixture, and without need for microscopic inspection or use of highly sensitive photometric equipment.

The method according to the present invention is particularly useful in blood grouping procedures. Yet it is also applicable to any other immunological test methods involving an antigen/antibody agglutination reaction. Such immunological methods include, e.g., blood testing methods, such as Rh-typing of blood, cross-matching of blood, Coombe's test, and antibody screening, e.g., of drugs such as antibiotics, test of bacteria, virus or protein suspensions.

Accordingly the terms "agglutinogen-containing" and "agglutinable matter" containing liquid suspension are used herein to denote not only cell suspension, e.g., blood and

blood cell suspensions but also suspensions of other proteineous or polymeric matter containing antigens or having agglutinogenic groups in their molecules, such as bacteria, latex or polymer suspensions.

The term "reagent" is used herein to denote not only antibody-containing liquids, such as antisera, but also test liquids which are susceptible of containing agglutinins or antibodies for a given agglutinable matter.

In a preferred embodiment, the method according to the present invention is used for blood grouping. The blood grouping procedure is carried out on a suspension of the red blood cells, which may be undiluted blood, an undiluted red cell containing fraction of the blood or a diluted red cell suspension having a red cell content of at least 2%, e.g., of from 2 to about 5%. Suitably an amount of from about 10 to about 150 microliters of the red cell suspension is used per test, preferably an amount corresponding to from about 10 to about 20 microliters of the undiluted red cell blood fraction. For example, if the test is carried out in a manual procedure it is advisable to use an amount of from 1 to about 5 drops, corresponding to from about 25 to 150 microliters of a diluted red cell suspension, whereas in an automated procedure preferably an amount of from about 10 to about 20 microliters of an undiluted red cell blood fraction is used. The blood grouping procedure comprises the steps of mixing a first sample of the red blood cell suspension with an effective amount of antiserum A, that is, an amount having a sufficient antibody content for agglutinating substantially the entire red cell content of the sample of red cell suspension in case these cells contain antigen A, e.g., an amount of from about 20 to 150 microliters, to obtain a first reaction mixture, mixing a second sample of the red blood cell suspension with an effective

amount of antiserum B to obtain a second reaction mixture, allowing the reaction mixtures to stand for a period of time sufficient for an immunological antigen/antibody reaction to take place effectively in the reaction mixture, e.g., a period of between about 1 and about 10 minutes, mixing each of the reaction mixtures with the biochemical agent, e.g., with an amount of between about 10 and 20 times its volume of the biochemical agent to obtain a first and second diluted reaction mixture, allowing a period of time sufficient for completion of the immunological reaction and settling out of agglutinated cells, e.g., a period of from about 1 minute up to about 25 minutes, preferably of between about 1 minute and about 5 minutes, and inspecting the diluted reaction mixtures visually or nephlometrically to determine whether non-agglutinated cells are retained suspended therein.

Referring now to the drawing, the apparatus for use in blood grouping consists essentially of a loading station 10, a first dispenser unit 11 for antisera, a delay station 12, an agitator unit 13, a second dispensing unit 14 for the biochemical agent, a stepwise progression unit 15, a reading unit 16, and an unloading station 17.

In use of the apparatus, racks 18 containing up to twelve test tubes 19 are fed manually to the loading station 10, and the racks are drawn to a position below spouts 20 of the dispenser unit 11 by means of a reciprocating consol 10A which carries opposed rows of spring-loaded pawls (not shown).

At the dispensing unit 11, similar quantities of different antisera are introduced simultaneously into the test tubes in rack 18A and, after a period of $1\frac{1}{4}$ minutes, the rack is moved to 18B at the delay station 12 by means of a reciprocating drive. The rack at 18B remains

in the delay station for a period of $1\frac{1}{4}$ minutes, and is then progressed to the agitator unit 13, where the rack, with its test tubes, is vibrated transversely of its length to effect thorough mixing.

After agitation for a period of 30 seconds, plus a delay of 45 seconds, the rack is moved to the second dispensing unit 14, where a movable spout 21 dispenses into each of the tubes identical quantities of the biochemical agent as hereinbefore described. A sensor may also be provided to prevent dispensing into an empty or absent tube. After the tubes are supplied with the agent, the rack is moved in four steps of $1\frac{1}{4}$ minutes duration to the reading unit 16. At the reading unit, a lever arm 22 carrying a laser-type beam emitter 23 directs successive beams of light through each of (in this case seven of) the twelve pairs of opposed openings 24 in the walls of the rack, and thus through the test tubes which contain the liquid solution. A sensor 25 simultaneously moves behind the rack to take photometric readings from the laser-type flashes which are of short duration. The sensor can also be adapted to monitor ambient light, and thus compensate for varying illumination conditions.

After the reading stage, the rack is moved to the unloading station 17 for manual removal.

The actuation and timing of the various (coordinated) operative steps may be effected by more-or-less conventionally electrically-operated triggers and drives, and the timing of the operative steps may be varied in accordance with test requirements. Also, heating or cooling means may be provided to standardise temparature.

The racks 18 are marked with bar code identifications which are also sensed at the reading unit. The results of the tests are then fed into a computerised analyser, and delivered in printed form, together with the identification of the blood sample, at print-out unit 26 on control panel 27.

The following example is intended to further illustrate the present invention without limiting its scope.

Example

A. Preparation of a biochemical agent for detecting immunological aggutination reactions:

700 ml of a solution of 1.42 g of sodium hydrogen phosphate ($Na_2HPO_4$) in 1 litre of 0.9% sodium chloride, and 300 ml of a solution of 1.36 g of potassium dihydrogen phosphate ($KH_2PO_4$) in 1 litre of 0.9% sodium chloride solution are mixed and the pH of the resulting buffered saline solution is adjusted to 7.2 at 20°C.

0.5 of Brij 35$^R$(polyoxyethylene-23-lauryl ether; manufacturer Atlas Chemical Industries), and 0.5 of Laureth sulphate(sodium laurylether sulfate containing 3-5 oxyethylene units per molecule) are added to 1 litre of the buffered saline solution and the mixture is stirred for 10 to 15 minutes by means of a magnetic stirrer.

B. Manual blood group procedure:

Samples of 2 drops of 2-4% red cell suspension in saline are introduced into three test tubes or cuvettes by means of an automatic dispenser. Into each test tube one drop of a different antiserum, that is, antiserum A, B, or AB, are added simultaneously. The reaction mixtures in the test tubes are allowed to stand for two minutes.

Then 1.5 ml of the biochemical agent are added to each of the tubes, and the diluted mixtures are allowed to stand for 5 minutes. Subsequently, the test tubes are examined visually by the naked eye to determine whether non-agglutinated cells are retained in suspension therein.

Additionally, the test tubes may be examined in a conventional nephlometer (light-scatter detector system) to determine whether or not they contain non-agglutinated cells suspended therein.

C. Automated blood grouping procedure.

10-microlitre samples of the undiluted red cell blood fraction of the blood to be grouped are introduced into each of at least seven test tubes 19 in a test tube rack 18 which is marked with identification bands. A succession of test tube racks are introduced into the loading station 10 of the apparatus, to be carried to the first dispensing unit 11 where five drops of the antisera are dispensed into each test tube. The test tube rack is then shifted through to the delay station 12, and the agitator unit 13, to the second dispensing unit 14 where an amount of 3 ml (± 10%) of the biochemical agent is dispensed into each of the test tubes. The rack is then transported to the reading unit 16 where a switch is tripped to activate the emitter 23 and the sensor 25 and also the sensor for reading the identification bands on the rack. Reading of the contents of each tube is thus effected precisely $8\frac{3}{4}$ minutes after the addition of the respective antiserum. The information from the sensing means is fed into a computer which is programmed to identify the blood group of the test sample based on this information and to print-out the result at the unit 26, in

conjunction with the patient's identification.

After the operation of the reading unit 16 is completed, the test tube rack is transported to the unloading station 17 where it is removed from the apparatus.

In the described apparatus, antisera is dispensed into seven test tubes containing blood samples, the antisera being anti-A, anti-B, anti-AB, anti-D(Rh), albumen control, A-cell, and B-cell, which produce a series of agglutination reactions sufficient to meet most blood grouping requirements. However, a further five control channels are included in the apparatus for testing up to an additional five blood samples, to provide further data on, for example, platelets.

As indicated above, the apparatus can be adapted for other procedures, such as testing latex or polymer suspensions or suspensions of bacteria.

CLAIMS

1. A biochemical agent for use in immunological analysis, substantially comprising from 0.1 to 1.0 g/l of a nonionic surface-active agent which is a water soluble protein-compatable polyoxyalkylene ether or ester, and from 0.1 to 1 g/l of a water soluble protein-compatable anionic detergent, in an aqueous isotonic buffered solution which has a pH value of from 6.5 to 8.0 and exhibits an osmolarity of from 300 to 400 milliosmol/kg, a sodium content of about from 125 to 225 milliequivalents/l, and a potassium content of from about 3.5 to 7.5 milliequivalents/l.

2. A biochemical agent as claimed in Claim 1, wherein the nonionic surface active agent is a polyoxyethylene ether of a $C_{12}$-$C_{16}$ alkyl alcohol containing from 20 to 25 oxyethylene units.

3. A biochemical agent as claimed in Claim 1, wherein the anionic detergent is a $C_{12}$-$C_{16}$ alkyl sulfate or a $C_{12}$-$C_{16}$ alkyl ether sulfate containing 3 to 5 oxyethylene units.

4. A biochemical agent, as claimed in any preceding Claim and substantially as hereinbefore described.

5. A method for detecting immunological agglutination in a liquid suspension, which comprises the steps of:

a) mixing a liquid suspension susceptible of comprising agglutinogen-containing agglutinable matter suspended therein with a reagent containing agglutinins reactive towards the agglutinogen-content of the agglutinable matter to obtain a reaction mixture;

b) allowing a period of time suitable for an immunological reaction to take place;

c) mixing the reaction mixture with a biochemical agent as claimed in any preceding Claim, to obtain a diluted reaction mixture; and,

d) examining the diluted reaction mixture for un-agglutinated subject matter suspended therein.

6. A method as claimed in Claim 5 and including the further step of agitating the mixture of liquid suspension and reagent.

7. A method as claimed in Claim 5 or Claim 6, for blood grouping, wherein step a) comprises mixing a red cell suspension, which is an undiluted or diluted red cell containing fraction of a blood sample, with an effective amount of an antiserum reagent.

8. A method as claimed in any of Claims 5 to 7 in which a series of different reagents as defined in step a), are mixed with a series of samples of the liquid suspension as defined in step a).

9. A method as claimed in any of Claims 5 to 8, wherein step b) is effected by passing a beam of light through the or each test tube and sensing the light transmitted through the test tubes.

10. A method as claimed in any of Claims 5 to 9 and including the further step of displaying or printing out the results of the detection.

11. A method of blood grouping, as claimed in any of Claims 5 to 10 and substantially as hereinbefore described.

12. An apparatus for the automated detection of immunological agglutination in a liquid suspension, the apparatus comprising:-

a) A loading station (10) for receiving a carrier (18) for a plurality of test tubes (19) or like receptacles, each tube containing a sample of the liquid suspension;

b) a first dispensing unit (11) for dispensing the required amount of reagent liquid into each of the test tubes in the carrier;

c) a second dispensing unit (14) for dispensing a metered amount of biochemical agent into each of the test tubes

d) a reading unit (16) comprising a photometric system (23) to pass beams of light through the test tubes, and light-sensitive means (25) for sensing the light consequently transmitted through the test tubes;

e) an unloading station (17) for discharging the test tube-containing carriers; and,

f) transport means for transporting the carriers from the loading station through the dispensing units, and the reading unit, to the unloading station.

13. An apparatus as claimed in Claim 12 and further comprising a mixing unit (13) located between the first and second dispensing units.

14. An apparatus as claimed in Claim 12 or Claim 13, and further comprising means (26) for printing out or displaying the results of the detection.

15. Apparatus for the automated detection of immunological agglutination in a liquid suspension, substantially as hereinbefore described with reference to the accompanying drawing.

0061541

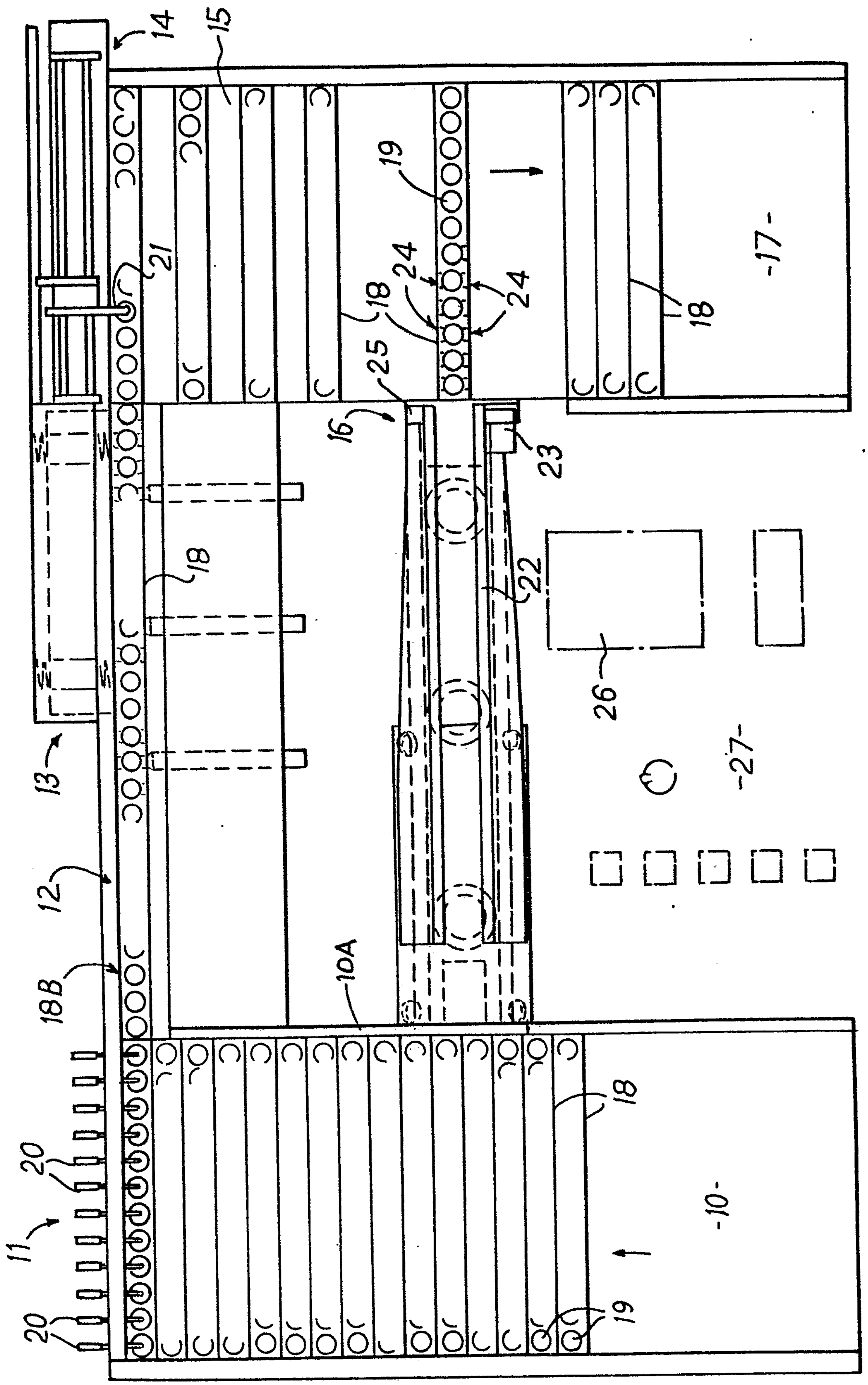

14
15
19
21
24
24
18
17
18
25
16
23
18
22
26
13
27
12
10A
18B
18
20
11
10
20
19

0061541

European Patent Office

# EUROPEAN SEARCH REPORT

Application number

EP 81 30 1341.4

| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| | DE - B1 - 2 816 229 (BOEHRINGER MANNHEIM)<br>* example 28 *<br>& US - A - 4 287 001<br>-- | 1-6 | G 01 N 33/50<br>G 01 N 33/80 |
| D | US - A - 4 130 395 (C.P. CHRYSSANTHOU)<br>* fig. 2, 3 *<br>-- | 12-15 | |
| A,D | US - A - 4 148 896 (D.H. SMITH, JR. et al.)<br>-- | | TECHNICAL FIELDS SEARCHED (Int. Cl.3) |
| A,D | US - A - 4 104 031 (R.G. ROBUCHON-MEROVAK et al.)<br>-- | | G 01 N 33/50 |
| A,D | US - A - 3 624 223 (W.J. SMYTHE)<br>---- | | |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| Berlin | 04-11-1981 | SCHWARTZ |

EPO Form 1503.1 06.78